Europäisches Patentamt

European Patent Office

Office européen des brevets

(11) Publication number: **0 163 623**
**B1**

## EUROPEAN PATENT SPECIFICATION

(45) Date of publication of patent specification: **09.05.90**

(21) Application number: **85850190.1**

(22) Date of filing: **30.05.85**

(51) Int. Cl.⁵: **C 12 P 21/00, A 61 K 37/02**

(54) The use of a cell surface protein obtained from Staph. aureus.

(30) Priority: **30.05.84 SE 8402938**

(43) Date of publication of application:
**04.12.85 Bulletin 85/49**

(45) Publication of the grant of the patent:
**09.05.90 Bulletin 90/19**

(84) Designated Contracting States:
**AT BE CH DE FR GB IT LI LU NL SE**

(56) References cited:
**EP-A-0 089 938**

**INFECTION AND IMMUNITY, vol. 37, no. 2, August 1982, pages 526-531 F. Espersen et al.: "Isolation of a fibronectin-binding protein from Staphylococcus aureus."**

**CHEMICAL ABSTRACTS, vol. 98, no, 17, April 25, 1983, page 222, ref. no. 139254c; Columbus, Ohio, US C.RYDEN et al.: "Fibronectin receptors from Staphylococcus aureus."**

(73) Proprietor: **ALFA-LAVAL AGRI INTERNATIONAL AB**
**Farm Center P.O. Box 39**
**S-147 00 Tumba (SE)**

(72) Inventor: **Höök, Magnus**
**4734 Bridge Water Road**
**Birmingham, Al. 35243 (US)**
Inventor: **Lindberg, Kjell Martin**
**Kornvägen 5**
**S-752 57 Uppsala (SE)**
Inventor: **Wadström, Torkel Mikael**
**P O Box 96**
**S-741 00 Knivsta (SE)**

(74) Representative: **Inger, Lars Ulf Bosson**
**L + U INGER Patentbyra HB Garvaregatan 12**
**S-262 00 Ängelholm (SE)**

Courier Press, Leamington Spa, England.

(56) References cited:
BIOLOGICAL ABSTRACTS, vol. 73, no. 5, 1982, pages 3311-3312, ref. no. 32144, Philadelphia, US K.H.SCHMIDT et al.: " Interaction of streptococcal cell wall components with fibrinogen. I. Adsorption of fibrinogen by immobilized T-proteins of Streptococcus pyogenes".

MEDLINE, Access numbers 1322045 (84162045) L.M.Switalski et al.: "Binding of Streptococcus pyogenes to laminin."

CHEMICAL ABSTRACTS, vol. 95, no. 3, July 20, 1981, page 216, ref. no. 19773n; Columbus, Ohio, US E.H.BEACHEY: "Bacterial adherence: Adhesin-receptor interactions mediating the attachment of bacteria to mucosal surfaces."

**Description**

Technical field

The present invention relates to the use of a cell surface protein having fibronectin binding properties.

The object of the present invention is to obtain a possibility of blocking fibronectin in a traumatic wound tissue in order to prevent adherence of pathogenic bacterial strains on fibronectin.

Background of the invention

Staphylococci and streptococci are usually often regarded as a group of gram positive bacteria, which develops purulent matter (pus) at infections, so called pathogenic cocci. This group does not only contain the classical *Staphylococcus aureus* and *Streptococcus pyrogenes* (group A streptococcus), but also other staphylococci and strepococci, such as *Staphylococcus epidermis, Staphylococcus haemolyticus, Staphylococcus hyicus,* streptococci of Groups B, C, G, and H, viridans streptococci, etc. Even gram negative bacteria such as *Escherichia coli* can cause such infections.

These pathogenic bacterial strains causes different infections in man and in animals all the way from small selfhealing skin infections, to serious sepsis (blood infection). At the infection of animals by these strains the animals are not only suffering, but also great economical damages are caused to the owners of the animals due to production cutoff. Mastitis in milking cows is such an economically damaging infection.

In man such bacterial strains cause i.a. heart valve infections, but also other infections as the commonly known "hospital illness", i.e., most often an infection of an open wound, which shows difficulties in healing, can produce large amounts of pus, and can cause reoperation. Particularly, the heart valve infections threatens risk groups already exposed within the hospital care.

The term wound used means that normally covering epithel cellular layer, and other surface structures have been damaged by mechanical, chemical, or other influence. The term wound can hereby be divided into two main groups, viz: surface wounds, and deep wounds. The term surface wound means a trauma on the surface of the body or a surface in direct connection to the cavities of the body, i.e., the gastro-intestinal duct, mouth cavity, urethra, milk ducts, etc. The term deep wounds means trauma in the inner of a body caused by violent outer assault or by surgical incisions in different tissues.

When a wound is caused, fibronectin, fibrinogen, collagen, and/or laminin are exposed in the wound tissue. These proteins form together with so called proteoglucans a net work structure in different reinforcement tissues, and is the structure onto which connective tissue (fibroblasts) and epithel cells grow at a natural wound healing.

The natural wound healing can, however, be prevented by pathogenic bacteria colonizing therein, primarily by pyogenic cocci, and secondly by other pathogenic strains, such as *E. coli* and other gram negative rod shaped bacteria.

Examples of such a colonizing of a tissue damage are:

i) colonizing of wounds in skin and connective tissue, which wounds have been caused by a mechanical violence, chemical damage, and/or thermical damage;

ii) colonizing of wounds on mucous membranes, such as in the mouth cavity, or in the mammalian glands, urethra, or vagina;

iii) colonizing on connective tissue proteins, which have been exposed by a minimal tissue damage (microlesion) in connection with epithel and endothel (mastitis, heart valve infection).

It is previously known from Infection and Immunity, *37,* (1982) pp. 526—531, Espersen, F. et al, Isolation of a fibronectin-binding protein from Staphylococcus aureus, to isolate fibronectin binding proteins from *Staph. aureus,* which proteins have a molecular weight of 197 000, and 60 000, respectively, whereby some concern over the properties of the smaller protein is expressed. The reference does not show that such a protein can be used in the treatment, prophylactic, or therapeutic, of wounds.

Description of the present invention

It has now surprisingly been shown possible to isolate proteins from *Staphylococcus aureus* cell surfaces, which proteins adhere to a fibronectin, which proteins have a molecular weight of between 40,000 and 165,000 and which can be used at the manufacture of a prophylactivally or therapeutically active wound treatment agent being active against wound pathogenic bacterial strains having fibronectin binding properties.

Such cell surface proteins can thereby be used for the treatment of wounds, e.g., for blocking protein receptors or for immunization (vaccination). In the latter case the body creates specific antibodies, which can protect against invasion of bacterial strains comprising such a cell surface protein. Hereby the antibodies block the adherence of the bacterial strains to a damaged tissue.

The characteristics of the present invention are evident from the accompanying claims.

By means of the present invention it is thus achieved that pathogenic bacterial strains can be effectively prevented from colonizing a traumatic wound tissue.

When using the present cell surface proteins for the purpose of immunization (vaccination) in mammals including man, the protein is dispersed in a sterile, isotonic saline solution, optionally while adding a pharmaceutically acceptable dispersing agent.

A suitable dosage to obtain immunization is 0.5 to 4 µg of cell surface proteins per kg bodyweight and injection of immunization. In order to obtain a durable immunization, vaccination should be carried out at three consecutive occasions with an interval of 1 to 3 weeks. Furthermore, the immunization shall be carried out in accordance with science and tested practise.

When using the present cell surface proteins for topical, local application the protein is dispersed in an isotonic saline solution to a concentration of 25 to 200 µg per ml. The wounds are then treated with such an amount only to obtain a complete wetting of the wound surface. For an average wound thus only a couple of millilitres of solution are used in this way. After treatment using the protein solution the wounds are suitably washed with isotonic saline solution or another suitable wound treatment solution.

Below, an immunization of young cows against mastitis is shown. Topical use of cell surface protein can also be used for preventing mastitis by treating udders/teats with a solution comprising cell surface proteins, which prevents pathogentic, mastitis-inducing organisms to adhere thereto.

In accordance with the invention a mixture of cell surface proteins with different binding properties can be used, particularly if the binding properties of an infecting, bacterial strain are unknown, and there is a great demand for a rapid prevention of a massive bacterial epidemic infection; or the infection is caused by a mixture of bacteria.

The invention will be described more in detail in the following with reference to some Examples.

Example

A strain of *Staphylococcus aureus,* which binds to fibronectin was grown on a liquid medium (TS-broth), trypticase-soya-extract (Oxoid, Ltd., England).

After finished growth the bacteria were isolated by centrifugation and were washed with a saline solution (0.9% NaCl in water). The bacteria were then decomposed using a bacteriolytic enzyme (Lysostraphin[R], Sigma, 5 mg/litre of cell culture). Fibronectin binding components were isolated by affinity chromatography on immobilized fibronectin bound to a dextrane gel (*Sepharose*[R], CL-4B, cyanobromide activated). The fibronectin binding components were the eluated by adding chaotropic ions (e.g. NaSCN, KSCN) in an aqueous solution. The eluation can also be carried out using an acidic solution, acetic acid solution having pH<3.

Fibronectin binding components consisting of proteins having their molecular weights within the range of 11,000 to 165,000, preferably 40,000 to 165,000 were isolated. The proteins may comprise a carbohydrate residue, whereby, however, it is the protein residue which is fibronectin binding, which is shown by the fact that the effect is totally eliminated after a treatment using protease, or heating to 80 to 100°C.

The amino acid composition of the protein components obtained is evident from the Table below:

TABLE

| Amino acid | Residues per 1000 amino acids | |
|---|---|---|
| | $M_w$=165K | $M_w$=87K |
| Aspartic acid | 146 | 134 |
| Threonine | 107 | 103 |
| Serine | 65 | 78 |
| Glutamine | 171 | 151 |
| Proline | 62 | 58 |
| Glycine | 79 | 84 |
| Alanine | 46 | 47 |
| Cysteine[a] | 2.3 | n.d. |
| Valine | 78 | 86 |
| Methionine[a] | 5.8 | n.d. |
| Isoleucine | 47 | 38 |
| Leucine | 40 | 46 |
| Tyrosine | 23 | 41 |
| Phenylalanine | 20 | 36 |
| Tryptophane[b] | 24 | 31 |
| Histidine | 32 | 30 |
| Lysine | 63 | 66 |
| Arginine | 12 | — |

[a] Amino acid determined after a performic acid oxidation of a sample.

[b] Amino acid calculated from an absorbance at 280 nm and tyrosine content.

n.d.=not determined.

In the Example the affinity chromatography has been used for purification/isolation of the protein. Other biochemical separation methods are ion exchange chromatography, and molecular sieve; electrophoresis incl. isotachophoresis; electrofocusing.

A conventional cultivation of *S. aureus* gives a cell surface protein of the above. For an efficient industrial production of receptors for vaccine, and other care the gen needs to be cloned in a suitable organism in order to obtain high yields.

A purified fibronectin binding cell surface protein has proved to be immunogenous at the immunization of rabbit and ruminants, and has thereby developed formation of antibodies.

Test 1

Vaccination of SRB-heifers (1:st calf cow) with a fibronectin binding protein in accordance with the Example above.

Three SRB-heifers (Swedish Red-and-White Cattle) were accinated subcutaneously in the thorax regon using 400 µg of fibronectin binding components ($M_w$ 165,000 and 87,000). These injections were repeated twice with 14 days inbetween. Antibody determinations in serum and in milk by means of ELISA-method (Enzyme Linked Immuno Sorbent Assay) showed a very potent immuno response determinable in large dilutions of milk and serum already at the moment for the second immunization.

Two weeks after the second injection, i.e., at the moment for the third immunization injection the immuno response was regarded as enough stimulated to carry out an experimental udder infection (mastitis) in the three animals. These three animals, as well as two control animals from the same stock were exposed to an experimental udder infection using a strongly udder pathogenic strain isolated from acute bovine mastitis (*S. aureus*) in order to develop mastitis in the five animals. The test was carried out by washing, dispersing in an isotonic saline solution and then injecting into the teat and udder cavity using a standardized injection technique, 500 bacteria from a bacterial cultivation grown in a broth medium (TS-broth).

The following results were obtained:

i) very sparse growth in certain milk samples from vaccinated cows, only;

ii) very high number of bacteria in most milk samples from non-vaccinated animals;

iii) cell count determinations showed generally low cell counts in the vaccinated animals;

iv) cell count determinations showed generally high cell counts in the non-vaccinated animals;

v) the vaccinated animals produced unchanged volumes of milk;

vi) the non-vaccinated animals showed markedly decreased milking volumes (>10%);

vii) determination of acute phase reactants type "C" reactive protein", and albumine in the vaccinated animals showed no change of the values obtained prior to the innoculation;

viii) determination of acute phase reactants type "C reactive protein", and albumine in the non-vaccinated animals showed strongly increased values.

The results obtained show that antibodies against fibronectin binding protein are secreted into udder and are present in local wound lesions in an amount enough to sterically preventing the surface receptors of an infecting bacterial strain to bind to exposed fibronectin in the udder tissue.

Test 2

Blocking of an infection in an open skin wound by wound treatment using fibronectin binding cell surface protein from *S. aureus.*

Standardized wound damages (2×2 cm) were made on the back of pigs (20—25 kgs) using a so called dermatom. These wounds placed in two rows of 8 wounds on each side of the spine were subjected to a thermical damage (250°C, 3 min). After thermical treatment the wounds were covered with a sterile bandage for 1.5 hrs, whereupon the wounds were infected with *S. aureus* strain (SA 113(83A)). Prior to bacterial infection the wounds on one side of the spine were treated with fibronectin binding cell surface protein, according to the Example above, solved in a sterile isotonic saline solution (100 µg per ml of NaCl-solution). In wounds pretreated in this way the development of an infection was prevented by, at the same time, washing the wounds twice a day using a sterile isotonic saline solution. Non-treated wounds showed in the lesions, bad infections within 2 to 4 days although washing twice a

day using NaCl-solution; infections which did not heal untreated with antibiotics during an observation period of one week.

The results of this experiment show that surface exposed fibronectin is blocked by pretreating lesions using 100 µg/ml in NaCl, in such a way that infections are prevented. Bacteria applied can easily be removed by rinsing which is impossible in wounds not treated with cell surface protein.

Test 3

The binding of Staphylococci to immobilized fibronectin—a model to simulate binding to traumatic tissue (surgical wounds and mastitis).

A polymer surface was treated with different serum prbteins, such as albumine and fibronectin. The polymer surface was then incubated with the respective protein dispersed in a sodium phosphate buffered saline solution (0.2 M sodium phosphate, pH 7.4, and 0.145 M NaCl) for 2 hrs at ambient temperature. The polymer surface was then dried by blowing air using a fan. Then the treated surface was subjected to a Staphylococci (strain SA 113(83A)) in a buffer solution, and dispersed in the presence of bovine milk, respectively. Already after a couple of minues an uptake of bacteria was determined in both these testing systems, while a surface treated in the same way using albumine in the same, and in a 10-fold higher concentration of protein solution does not show an active bacterial uptake (untreated surface is however hydrophobic and binds staphylococci unspecific). The binding of strain SA 113(83A) can be inhibited by first incubating the bacteria with an antiserum obtained from rabbit vaccinated with a purified receptor protein.

Test 4

A polymer surface was treated with fibronectin (immobilized in accordance with Test 3 above. Then the surface was treated with a cell surface protein ($M_w$ 87,000) of Example 1 above solved in a physiological saline solution, 100 µg per ml. Then the surface was treated with a Staphylococci (strain SA 113(83A)) dispersed in a buffer solution (phosphate buffer, 0.2 M Na-phosphate, pH 7.4, and 0.145 NaCl). After the treatment with staphylococci the polymer surface was rinsed with a physiological saline solution for eliminating loosly attached bacteria. At a subsequent analysis it was determined that no active binding of the staphylococci had taken place. The analysis was carried out by determining bacterial cell mass ATP (adenosine triphosphate) by means of bioluminiscens technique. In short the analysis is carried out by incubating the polymer surface with 50 µl of 1.25 N trichloro acetic acid to extract cellular ATP. The amount of ATP is determined and compared with a standard curve for ATP in a Luminometer 1250 (LKB-Produkter, Bromma, Sweden).

**Claims**

1. The use of a cell surface protein from *Staph.*

*aureus* having fibronectin binding properties and having a molecular weight of between 40,000 and 165,000 at the manufacture of a prophylatically or therapeutically active, wound treatment agent being active against wound pathogenic bacterial strains having fibronectin binding properties.

2. The use according to claim 1, wherein an immunizing wound treatment agent is manufactured comprising an immunizing amount of said cell surface protein.

3. The use according to claim 1, wherein a wound treatment agent in the form of a topically applicable agent comprising a prophylactical therapeutically active amount of said cell surface protein is manufactured.

4. The use according to claims 1 and 2, wherein an injectable wound treatment agent for the treatment of mastitis in ruminants is manufactured.

5. The use according to claims 1 and 2, wherein an injectable wound treatment agent for the treatment of bacterial infections in humans is manufactured.

**Patentansprüche**

1. Verwendung eines Zelloberflächenproteins aus Staph. aureus mit Fibronectin bindenden Eigenschaften und mit einem Molekulargewicht zwischen 40 000 und 165 000 bei der Herstellung eines prophylaktisch oder therapeutisch aktiven Wundbehandlungsmittels, das gegen pathogene Wundbakterienstämme mit Fibronectin bindenden Eigenschaften aktiv ist.

2. Verwendung nach Anspruch 1, bei der ein immunisierendes Wundbehandlungsmittel mit einer immunisierenden Menge des Zelloberflächenproteins hergestellt wird.

3. Verwendung nach Anspruch 1, bei der ein Wundbehandlungsmittel in der Form eines örtlich aufbringbaren Mittels mit einer prophylaktischen therapeutisch wirksamen Menge des Zelloberflächenproteins hergestellt wird.

4. Verwendung nach den Ansprüchen 1 und 2, bei der ein injizierbares Wundbehandlungsmittel für die Behandlungs von Mastitis bei Wiederkäuern hergestellt wird.

5. Verwendung nach den Ansprüchen 1 und 2, bei der ein injizierbares Wundbehandlungsmittel für die Behandlung bakterieller Infektionen bei Menschen hergestellt wird.

**Revendications**

1. Utilisation d'une protéine de surface cellulaire provenant de *Staphylococcus aureus*, ayant des propriétés de liaison à la fibronectine et présentant un poids moléculaire compris entre 40.000 et 165.000, dans la fabrication d'un agent de traitement des plaies, actif du point de vue prophylactique ou thérapeutique, et actif contre les souches bactériennes pathogènes des plaies, cet agent présentant des propriétés de liaison à la fibronectine.

2. Utilisation suivant la revendication 1, dans laquelle un agent de traitement immunisant pour les plaies est fabriqué en comprenant une quantité d'immunisation de la protéine de surface cellulaire précitée.

3. Utilisation suivant la revendication 1, dans laquelle on fabrique un agent de traitement des plaies sous forme d'un agent applicable topiquement, comprenant une quantité active du point de vue prophylactique et du point de vue thérapeutique de la protéine de surface cellulaire précitée.

4. Utilisation suivant les revendications 1 et 2, dans laquelle on fabrique un agent de traitement injectable pour les plaies en vue du traitement de la mastite chez les ruminants.

5. Utilisation suivant les revendications 1 et 2, dans laquelle on fabrique un agent de traitement injectable pour les plaies en vue du traitement des infections bactériennes chez les êtres humains.